(19) Europäisches Patentamt
European Patent Office
Office européen des brevets

(11) **EP 1 465 535 B1**

(12) **EUROPEAN PATENT SPECIFICATION**

(45) Date of publication and mention
of the grant of the patent:
**28.11.2007 Bulletin 2007/48**

(51) Int Cl.:
*A61B 17/20* (2006.01)   *A61M 37/00* (2006.01)
*A61N 1/30* (2006.01)

(21) Application number: **02805673.7**

(22) Date of filing: **20.12.2002**

(86) International application number:
**PCT/US2002/041208**

(87) International publication number:
**WO 2003/053258 (03.07.2003 Gazette 2003/27)**

(54) **SKIN-PIERCING MICROPROJECTIONS HAVING PIERCING DEPTH CONTROL**

MIKROVORSPRÜNGE ZUM DURCHSTECHEN DER HAUT MIT STECHTIEFENSTEUERUNG

MICROPROJECTIONS POUR LE PERCAGE DE LA PEAU A REGULATION DE LA PROFONDEUR DE PERCAGE

(84) Designated Contracting States:
**AT BE BG CH CY CZ DE DK EE ES FI FR GB GR IE IT LI LU MC NL PT SE SI SK TR**

(30) Priority: **20.12.2001 US 342553 P**

(43) Date of publication of application:
**13.10.2004 Bulletin 2004/42**

(73) Proprietor: **ALZA CORPORATION Mountain View, CA 94039-7210 (US)**

(72) Inventors:
• **TRAUTMAN, Joseph, C.**
  **Sunnyvale, CA 94087 (US)**
• **CORMIER, Michel, J., N.**
  **Mountain View, CA 94043 (US)**

(74) Representative: **Mercer, Christopher Paul et al Carpmaels & Ransford 43-45 Bloomsbury Square London WC1A 2RA (GB)**

(56) References cited:
**WO-A-97/48440         GB-A- 2 354 446 US-A- 5 820 562**

**Description**

**TECHNICAL FIELD**

The present invention relates to transdermal agent delivery and more particularly, to the transdermal delivery of drugs and vaccines and/or transdermal sampling of body analytes such as glucose. More particularly the invention relates to a device having a plurality of stratum corneum piercing microprojections which when applied to the skin, pierce to an predetermined uniform depth of penetration, thereby providing optimum results for agent delivery and/or sampling.

**BACKGROUND ART**

Devices used for cutting skin, e.g. surgical scalpels and the like, have been known and used for sometime. See for example, MacKool U.S. Patent 5,810,857. In addition, devices used for piercing the skin, e.g., using pointed knives that are pushed into the skin, are also known for applications such as surgically implanting hair plugs. See for example, Ashraf, U.S. Patent No. 6,197,039. Devices of this type are intended to make relatively deep cuts. In the case of surgical knives, cuts have a depth measuring in centimeters and in the case of pointed knives used for implanting hair elements, depths are of at least 0.5 centimeters. Such devices are well outside the scope of the present invention. The present invention utilizes microprojection arrays designed to be minimally invasive, generally penetrating the skin to depths of less than 0.5 mm.

Such micro-penetrating devices have been disclosed for example, in Daddona et al., U.S. Patent No. 6,091,975, Cormier et al., U.S. Patents Nos. 6,219,574 and 6,230,051 and in Godshall et al., U.S. Patent No. 5,879,326. All of these devices disclose tiny microprojections extending from a base sheet or substrate and having lengths generally less than 0.5 mm. Godshall et al., ('326) further discloses that the base plate acts as a stop for preventing the microprojections from penetrating the skin beyond a predetermined distance.

More recently, it has been discovered that due to the elastic/rubbery nature of human skin, these types of microprojection arrays tend to have wide variability in depth of penetration from one microprojection to the next. Furthermore, because of the viscoelastic nature of skin, skin tends to dimple down in the areas of the skin surrounding the piercing microprojections with the result that the microprojection does not pierce to a depth that is equal to the microprojection length. The amount of skin dimpling can be lessened by stretching the skin at the time of microprojection piercing. See for example Trautman, et al., WO 01/41863. Unfortunately, even skin stretching does not completely compensate for the skin dimpling property and the resulting partial and variable penetration of the microprojections. While one potential solution is to use longer microprojections, because of the inherent variability in penetration depths of the devices and the piercing techniques used to date, longer microprojections have inevitably resulted in some portion of them penetrating the skin too deeply, with the attendant undesirable result of bleeding and in some cases, discomfort to the patient. US 5,820,562 describes a skin allergy test bar comprising a plurality of step-shaped punctures and a raised portion, which act as a stopping mechanism to prevent the punctures penetrating the skin too deeply,

DISCLOSURE OF THE INVENTION

The present invention provides a device for forming a plurality of microcuts in animal skin as defined in the appended set of claims, the microcuts having a predetermined depth of penetration of less than 500 microns. The device includes a member having a plurality of skin-piercing microprojections extending therefrom. Each of the microprojections has a base, a tip, an edge, a face and a length that is measured from the base to the tip. The length of the microprojections are substantially longer than the predetermined depth of penetration. The device has a piercing depth limiter associated with at least a portion of the microprojections. The piercing depth limiter is positioned at a predetermined location between the tip and the base of the microprojection whereby the limiter greatly reduces the tendency of the microprojection to pierce the skin beyond the predetermined depth.

According to one embodiment of the invention, at least a portion of the skin piercing microprojections, preferably at least about 10% of the microprojections, and most preferably substantially all of the skin-piercing microprojections, have a piercing depth limiter in the form of a skin surface abutting surface. The reference to the skin surface abutting surface refers to the fact that this surface is positioned directly on top of the skin after the microprojections have been applied to the skin. In one preferred embodiment, this surface comprises one or a plurality of shoulders adjacent to the microprojection tip which ensures that only the tip penetrates through the skin and not the remaining portions of the microprojection length.

The microprojection device is comprised of a sheet, e.g., a metal sheet, having a plurality of openings therein with the microprojections extending from the sheet adjacent to the openings. In a preferred embodiment, the limiter comprises a member having a plurality of stop protrusions which member is adapted to be positioned adjacent the skin distal side of the sheet with the microprojections and openings. In this embodiment, the sheet and the member are positioned adjacent one another so that the stop protrusions extend through at least a portion of, and preferably through substantially all of, the openings in the sheet.

The invention will now be described in connection with certain preferred embodiments which are illustrated in the figures and disclosed hereinafter.

## BRIEF DESCRIPTION OF THE DRAWINGS

A better understanding of the present invention as well as other objects and advantages thereof will become apparent upon consideration of the following detailed description especially when taken with the accompanying drawings, wherein like numerals designate like parts throughout, and wherein:

Figure 1 is a top perspective view of a portion of a microprojection array as is known in the prior art;

Figure 2 is a side view of a single microprojection piercing through skin in accordance with one embodiment of the present invention;

Figure 3 is a perspective view of the microprojection shown in Figure 2;

Figure 4 is a perspective view of a portion of a microprojection array having microprojections of the type shown in Figures 2 and 3;

Figure 5 is a perspective view of a single microprojection in accordance with one embodiment of the present invention;

Figure 6 is a perspective view of a microprojection array having microprojections of the type shown in Figure 5;

Figure 7 is a perspective view of a single microprojection in accordance with another embodiment of the present invention;

Figure 8 is a perspective view of a microprojection array having microprojections of the type shown in Figure 7;

Figure 9 is a perspective view of a single microprojection in accordance with another embodiment of the present invention;

Figure 10 is a side sectional exploded view of an unassembled alternative embodiment of a microprojection penetration stop mechanism in accordance with the present invention;

Figure 11 is a side sectional view of the device shown in Figure 10 illustrating of the device in an assembled condition;

Figure 12 is a top view of a single microprojection in accordance with another embodiment of the invention; and

Figure 13 is a top view of a single microprojection in accordance with yet another embodiment of the invention.

**DETAILED DESCRIPTION OF PREFERRED EMBODIMENTS**

The device of the present invention more consistently, uniformly, and reliably penetrates a body surface, e.g. the outermost stratum corneum layer of skin, to enhance agent delivery and/or body analyte sampling therethrough. The device of the present invention achieves greater uniformity in the depth of penetration from one microprojection to the next, and a decreased chance of microprojection piercing being so deep as to cause bleeding and/or discomfort. As used herein, the term "microprojections" refers to very tiny skin piercing elements, typically having a length of less than 500 $\mu$m, a width of less that 400 $\mu$m and a thickness of 5 to 100 $\mu$m which make correspondingly sized microcuts/ microslits in the skin. Upon piercing through the outermost layer (i.e., the stratum corneum) of the skin, the microprojections form pathways through which an agent such as a drug can be introduced, i.e. transdermally delivered, and/or through which a body analyte such as glucose can be sampled by collection of body fluids, optionally stored within a reservoir associated with the microprojections. For agent delivery, the agent may be incorporated in a separate reservoir associated with one or more microprojections or the agent may be incorporated as a coating on the microprojections and/or other portions of the device. An important feature of the present invention is a microprojection device with the microprojections having lengths which are intentionally selected to be substantially longer than the desired depth of penetration. In addition, the device has a piercing depth limiter, which is on, is part of one or more microprojection or is closely associated one or more microprojections, which substantially reduces bleeding caused by the microprojections piercing too deeply into the skin. The limiter also allows for a more consistent amount of agent delivery or sampling due to a more uniform depth of microprojection penetration. Furthermore, the present invention reproducibly provides greater uniformity in microprojection penetration from patient to patient and from one microprojection to the next in a single microprojection array applied to a single patient.

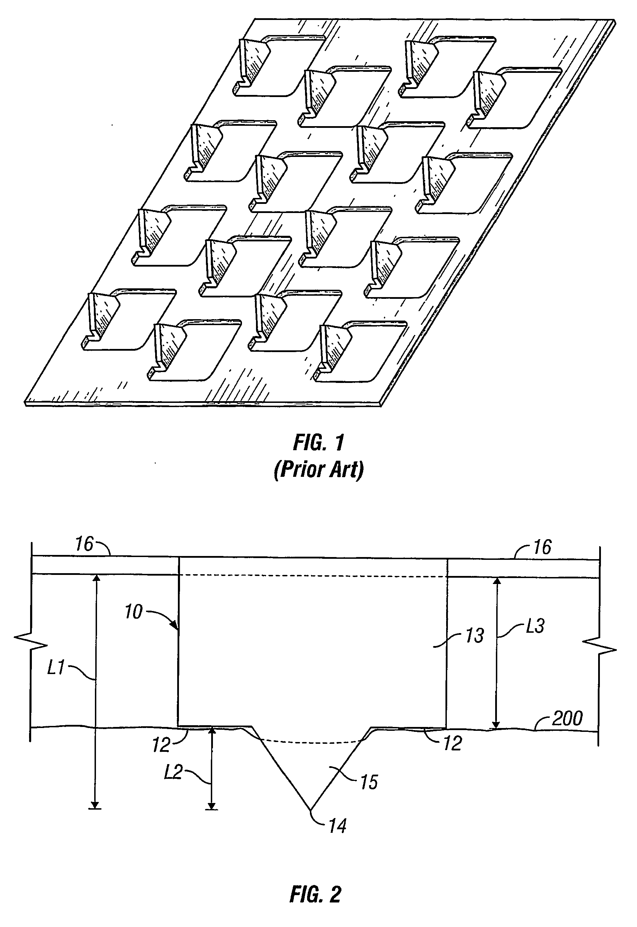

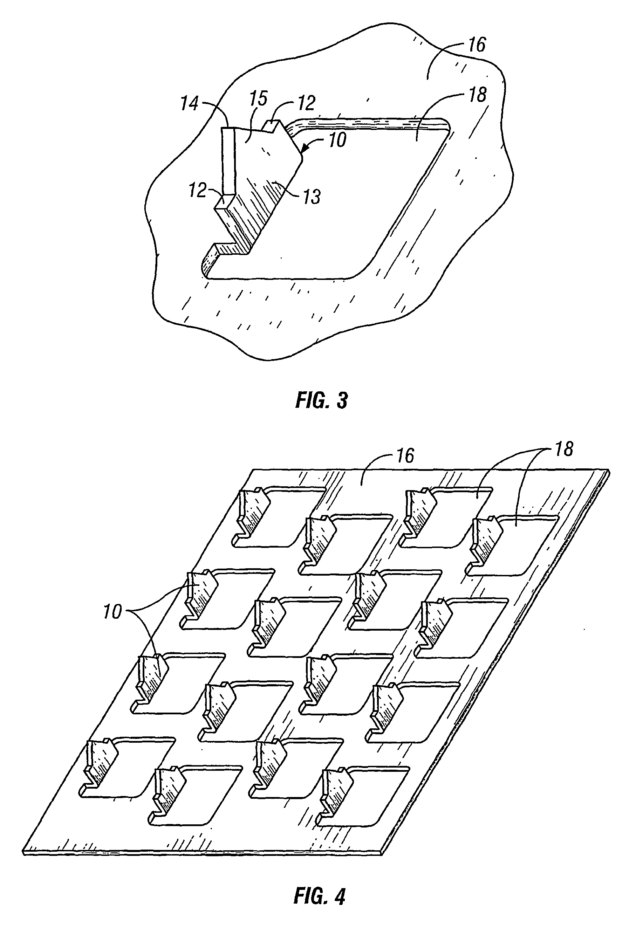

Fig. 1 illustrates a prior art microprojection array without the piercing depth limiter of the present invention. This can be contrasted with the device shown in Fig. 4 having a piercing depth limiter in accordance with one embodiment of the present invention.

These microprojection arrays are typically formed from a sheet wherein the microprojections are formed by etching or punching the sheet and then the microprojections are folded or bent out of a plane of the sheet

Referring now to Figs. 2 through 4, a plurality of microprojections 10 extend from a sheet 16. The microprojection 10 are located around the periphery of openings 18. The microprojections 10 have a penetrating portion 15, a non-penetrating portion 13, and two shoulder-like limiters 12. The limiters 12 are located a predetermined length L2 from the end 14 of penetrating portion 15. The overall length L1 of the microprojection 10 is substantially longer than the length L2 of the penetrating portion 15. Although the invention is not limited to any particular values for the ratio of L2:L1, for microprojections having an L2 between about 50 microns and about 400 microns, the ratio of L2:L1 is typically less than about 0.5. This ratio will also be effected by the particular conditions of microprojection penetration, including the microprojection density (I.e., the number of microprojections per cm$^2$ of the array), the tautness of the skin and the piercing force applied to the array. What is important is to ensure that the length of the non-penetrating portion 13 (I.e., L3 which is equal to L1 minus L2) Is sufficiently long to compensate for the dimpling property of skin and to allow the penetrating portion 15 to pierce completely into the skin 200 with the limiters 12 abutting against the surface of skin 200. Thus, the distance L2 from the end 14 of the microprojection 10 to the limiter 12 is substantially equal to the depth of penetration into skin 200.

The leading edge of the projection may have a sharp arrowhead-like edge or a sloping angle point to cut or Incise the skin 200. Thus, the limiter 12 is designed to inhibit the microprojection 10 from penetrating any deeper than the predetermined length L2. When the array is Impacted against the skin 200 using a spring loaded impactor of the type disclosed in Cormier et al., WO 02/30309A1, published 18 April, 2002, the microprojections 10 pierce the skin 200 to the point where the limiters 12 abut against the skin surface and retard further penetration Into the skin 200. The limiters 12 may be located on both sides of penetrating portion 15 as shown In Figs. 2-4..

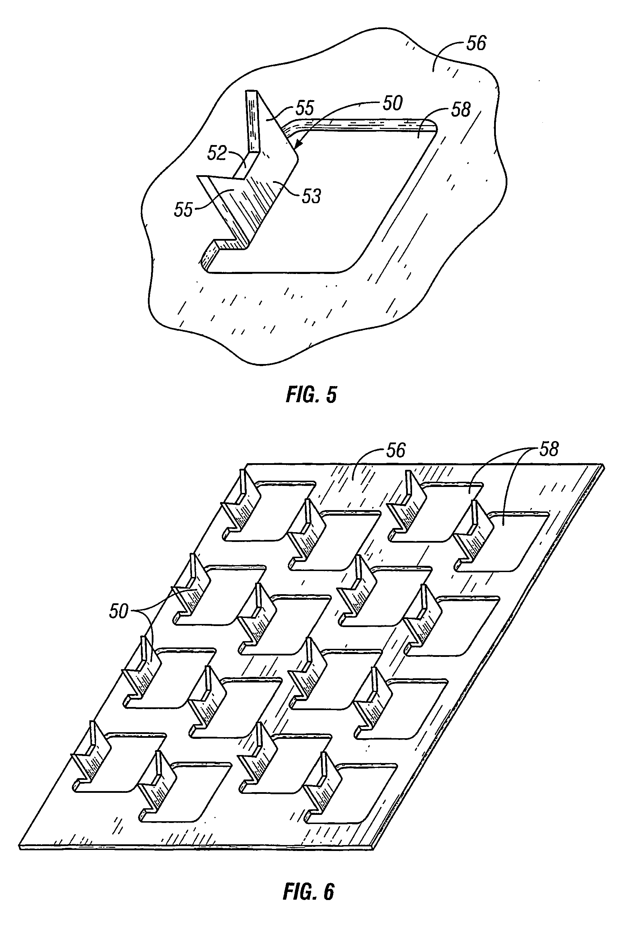

Alternatively, the limiter may be position between a pair of penetrating portions. One such embodiment is shown in Figs. 5 and 6. A plurality of microprojections 50 are shown located around a plurality of openings 58 in sheet 56. In this embodiment, limiter 52 is positioned between a pair of penetrating portions 55. Though this and other embodiments of the invention disclosed herein provide for a single microprojection 50 extending from a single opening 58, it is within the scope of the present invention that microprojection arrays may include one or more microprojections locate around the periphery of each opening.

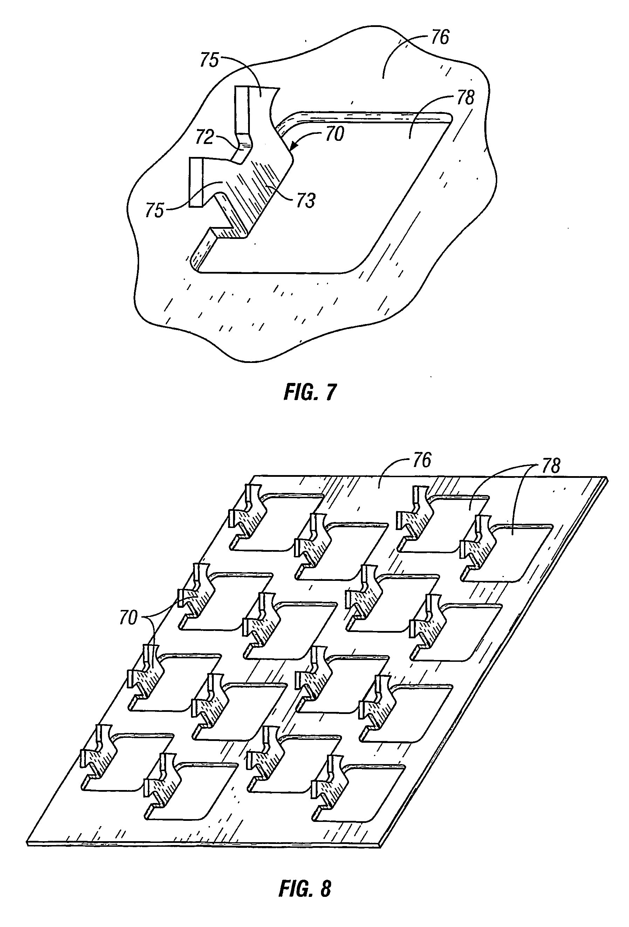

Another such embodiment is shown in Figs. 7 and 8. A plurality of microprojections 70 are shown located around a plurality of openings 78 in sheet 76. In this embodiment, limiter 72 is positioned between a pair of penetrating portions 75 that have a different shape than penetrating portions 55. The effect of limiter 72 is to limit the penetration of microprojections 70 to the penetrating portions 75 while the non-penetrating portion 73 remains outside the skin.

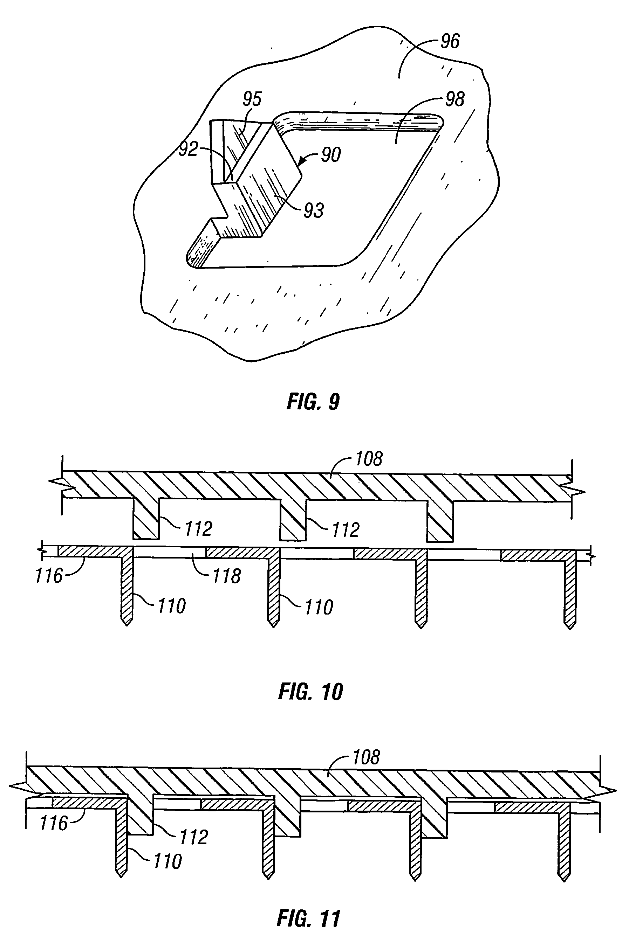

Yet another piercing depth limiter design is shown in Fig. 9. In this embodiment, the thickness of sheet 96 is intentionally selected to be substantially greater than the desired thickness of penetrating portion 95. The penetrating portion 95 is then subjected to additional acid etching to form a limiter surface 92 between the penetrating portion 95 and the non-penetrating portion 93.

Referring now to Figs. 10 and 11, there is shown an alternate embodiment of a microprojection piercing depth limiter in accordance with the present invention. In this embodiment, the limiter is a separate element from the microprojection array and hence can be used with microprojection arrays of the type illustrated in Fig. 1. In this embodiment, the limiter is shown as stop member 108 in the form of a layer having a plurality of stop protrusions 112 extending therefrom. The stop protrusions 112 are sized and spaced so as to extend through the openings in the sheet 116 of the microprojection array. Fig. 10 shows the sheet 116 and the stop member 108 prior to assembly whereas, Fig. 11 shows the two members after assembly and ready for use. If desired, small quantities of adhesive can be used in order to ensure that the sheet 116 and member 108 remain secured to one another. Member 108 can be composed of metals, ceramics, plastics and other suitable materials. Though stop protrusion 112 are not physically attached to or part of the microprojections, they function in the same manner as shown in the previous embodiments by controlling and limiting the dept of the penetration portion of the

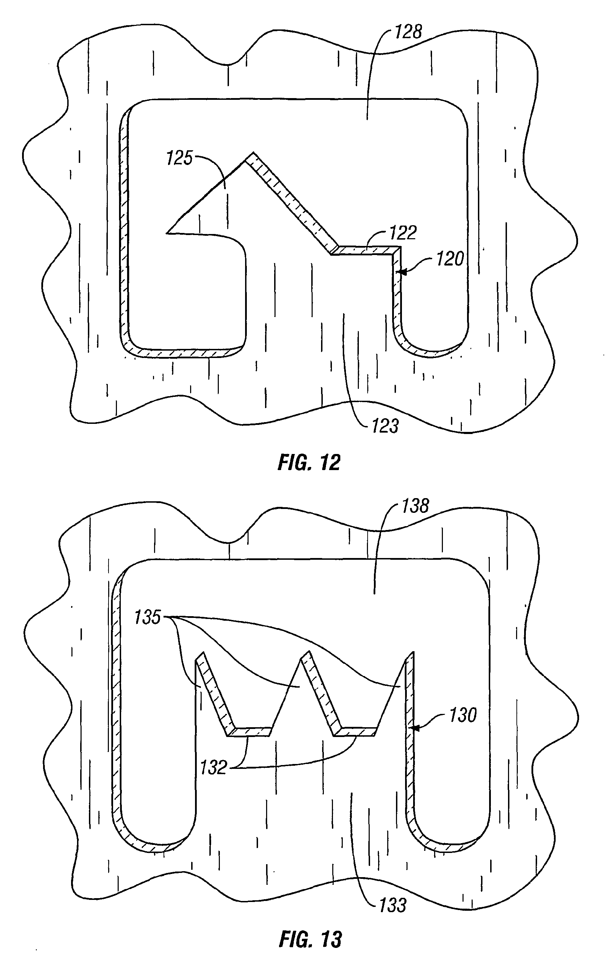

Additional embodiments of microprojection and limiter design are shown in Figures 12 and 13. Figure 12 shows microprojection 120 located along the periphery of opening 128. Microprojection 120 has a penetrating portion 125, a limiter 122 and a non-penetrating portion 123. This figure shows the microprojection after it has been formed out of the sheet and prior to being bent out of a plane of the sheet.

Figure 13 shows microprojection 130 located along the periphery of opening 138. Microprojection 130 has three penetrating portions 135, two limiters 132 and a non-penetrating portion 133. This figure shows the microprojection after it has been formed out of the sheet and prior to being bent out of a plane of the sheet.

In general, the limiters or "stops" are step like skin surface abutting surfaces that extend horizontally from either the narrow edge (e.g. see fig. 2-4) or the wider face of a microprojection (e.g. see fig. 9). Each stop extends horizontally at the point of the predetermined length of the preferred penetration depth. At this point an extension perpendicular to the direction of penetration extends from the microprojection. These stops inhibit, and preferably substantially prevents, penetration of the microprojections deeper than the location of the stops. The stops can be configured in various ways in relation to the microprojection with which it is associated. For example, the stops can be on both sides of the penetrating portion, in between multiple penetrating portions, on only one side of the base of the penetrating portion, and/or perpendicular to the face of the penetrating portion. The width of each stop section should be wide enough to inhibit, and preferably substantially prevent, the penetrating portion from entering the material any further than the predetermined length. When the stop is formed by a horizontal extension from the narrow edge of the microprojection, the stop is of the same thickness as each microprojection and the sheet from which the microprojections were formed.

By incorporating the limiter or stops on the microprojections, the undesired effects of the penetration being too deep and causing unwanted pain and bleeding are reduced, and preferably are substantially eliminated. Each stop lies approximately parallel to the surface or material being penetrated, therefore retarding further penetration. The number of stops can vary. It is not required that there be a stop adjacent to each microprojection within the array. Preferably at least about 10% of the microprojections have a stop or limiter closely adjacent thereto and most preferably substantially all of the microprojections have a stop or limiter closely adjacent thereto.

In addition to penetration of the microprojection, the preferred application device provides bi-directional stretching of the skin. The skin is stretched from two directions as the applicator is pressed against the skins surface. Thus allowing a more uniform penetration e.g. generates the same size and depth pathways, by the microprojection. When piercing the skin with very tiny microprojections the degree of tension under which the skin is placed becomes more critical compared to skin piercing using substantially larger piercing elements. The applicator for the sheet of microprojections of the present invention may take on different shapes. The present invention can be used with any known application device and is not limited to any particular application device.

Also within the present invention, there is no particular shape or form that is required for the microprojections. Within the preferred embodiment, each microprojection will include a sloped angle or arrowhead like pointed tip that allows incising the material (skin) more easily. The microprojection in its entirety can have one or multiple penetrating peaks or edges and one or more skin-abutting depth limiting surfaces variously configured.

Other advantages of the present invention are further illustrated by the following examples.

**Example 1**

A study was performed to assess the uniformity of microprojection penetration through excised hairless guinea pig skin. Microprojection arrays of the type illustrated in Fig. 7 were applied to excised hairless guinea pig skin using a spring loaded impact device of the type illustrated in Fig. 1 of Cormier, et al., WO 02/30301A1. This device supplied an impact of about 0.05 Joules/$cm^2$. The microprojection dimensions were as follows:

$$L1 = 204 \text{ microns}$$

$$L2 = \quad 75 \text{ microns}$$

Microprojection density: 348 microprojections per $cm^2$

The patches were removed following impact application and the skin sites were stained with India ink. The sites were biopsied and sliced parallel to the surface of the skin using a cryotome to measure the depth of penetration of the ink.

The penetration depth in his study did not exceed 60 microns and exhibited acceptable depth variability.

**Comparative Example 1**

A study similar to that described in Example 1 is performed with a microprojection array having microprojections of the shape illustrated in Fig. 1, i.e., without any piercing depth limiting feature. The microprojections had the following dimensions:

$$L1 = 241 \text{ microns}$$

Microprojection density: 321 microprojections per $cm^2$

The same impact conditions were used as in Example 1. After performing a biopsy and slicing the skin, the maximum penetration depth is found to be 140 microns with a greater variability in penetration depth than that seen in Example 1.

**Example 2**

A study similar to that described in Example 1 and Comparative Example 1 was performed with titanium sheet microprojection arrays having a circular shape and a skin contact area of 2 $cm^2$. The skin contact area being the area enclosed by the periphery of the circular array. The arrays were fastened to adhesive overlays having an area of 5 cm². The patches (i.e., array plus overlay) were applied to excised hairless guinea pig skin by an impact applicator having an impact energy of 0.053 joules/cm² and a hold down force of 0.44 Newtons. The skin sites were stained, biopsied and sliced as in Example 1. The results are shown in Table 1.

**TABLE 1**

| Microprojection Design | L1 ($\mu$m) | L2 ($\mu$m) | Microprojection Density (/cm²) | Average Penetration Depth ($\mu$m) | Standard Deviation In Penetration Depth ($\mu$m) |
|---|---|---|---|---|---|
| FIG. 12 | 206 | 116 | 348 | 64 | 20 |
| FIG. 1 | 197 | N/A | 348 | 58 | 22 |

**Claims**

1. A device for forming a plurality of microcuts in animal skin, the microcuts having a predetermined depth of penetration, said device comprising:

   a) a first member having a plurality of skin piercing microprojections (10) extending therefrom, comprising a sheet (16) having a skin proximal surface and a skin distal surface, the sheet (16) having a multiplicity of openings (18) therein and the microprojections (10) extend from a body proximal surface of the sheet (16), the microprojections (10) being adapted to pierce the skin to a predetermined depth of penetration of less than about 500 microns;
   b) each of the microprojections (10) comprising a base, a penetrating portion (15) having a length L2, a tip (14), an edge, a face and a length, L1, that is a distance from the base to the tip (14) of the microprojection, said length being substantially longer than said predetermined depth;
   and **characterised in that**:
   c) a piercing depth limiter (12) is associated with one or more of the microprojections (10) and positioned at a predetermined location between the tip (15) and the base, wherein the limiter (12) restricts the piercing of the microprojections (10) to about the predetermined depth.

2. The device of claim 1, wherein the limiter (12) comprises a skin-abutting surface.

3. The device of claim 2, wherein the skin-abutting surface of the limiter (12) is approximately parallel to the skin surface at the time the device penetrates the skin.

4. The device of claim 2, wherein the distance between a tip (14) of the microprojection (10) and the skin-abutting surface of the limiter (12) substantially equals the predetermined depth.

5. The device of claim 1, wherein the limiter (12) is an integral structure of one or more of said microprojections (10).

6. The device of claim 5, wherein the limiter (12) is an integral structure of each microprojection (10).

7. The device of claim 6, wherein each microprojection (10) has a plurality of limiters (12).

8. The device of claim 5, wherein the limiter (12) is a skin-abutting surface.

9. The device of claim 8, wherein at least 10% of the microprojections (10) have at least one limiter (12).

10. The device of claim 1, wherein the limiter (12) comprises a shoulder extending from said penetrating portion (15) of one or more of said microprojections (10).

11. The device of claim 8, wherein substantially all microprojections (10) have at least one limiter (12).

12. The device of claim 1, wherein the device includes an agent-containing or agent-receiving reservoir.

**13.** The device of claim 12, wherein the reservoir is in agent-transmitting relation with the openings (18) in the sheet (16).

**14.** The device of claim 1, wherein the first member comprises a sheet (16) having a multiplicity of openings (18) therein and the microprojections (10) extend from a body proximal surface of the sheet (16).

**15.** The device of claim 14, wherein the limiter comprises a second member (108) having a multiplicity of stop protrusions (112), the limiter being positioned adjacent the skin distal side of the sheet (116) with the protrusions (112) being appropriately spaced to extend into the openings (118) of the sheet (116), the stop protrusions (112) extending a predetermined distance through the openings (118) in the sheet (116).

**16.** The device of claim 15, wherein the microprojections (110) are positioned adjacent the openings (118) through which the stop protrusions (112) extend.

**17.** The device of claim 15, wherein the microprojection (110) length minus the predetermined distance of protrusion (112) extension substantially equals the predetermined depth of penetration.

**18.** The device of claim 15, wherein the member (108) having a multiplicity of stop protrusions (112) is comprised of a material selected from the group consisting of plastic, elastomer and rubber.

**19.** The device of claim 1, wherein the ratio of L2 to L1 is less than about 0.5.

**20.** The device of claim 14, wherein the thickness of said penetrating portion (15) of one or more microprojections (10) is less than the thickness of the sheet (16) and said limiter (12) comprises one or more skin abutting surfaces associated with one or more microprojections (10).

**21.** The device of claim 20, wherein the width of the sheet (16) minus the width of the penetrating portion (15) is about the same as the width of the limiter (12).


**Patentansprüche**

**1.** Vorrichtung zum Ausbilden einer Mehrzahl an Mikroschnitten in Tierhaut, wobei die Mikroschnitte eine vorgegebene Eindringtiefe haben, wobei die Vorrichtung umfaßt:

a) ein erstes eine Platte (16) mit einer hautseitigen Oberfläche und einer hautabgewandten Oberfläche umfassendes Element mit einer Mehrzahl an davon ausgezogenen hautdurchstechenden Mikroprojektoren (10), wobei die Platte (16) eine Vielzahl an Öffnungen (18) darin aufweist und die Mikroprojektoren (10) aus einer körperzugewandten Oberfläche des Blatts (16) ausgezogen sind, dabei wurden die Mikroprojektoren (10) angepaßt, um die Haut bis zu einer vorbestimmten Eindringtiefe von weniger als ungefähr 500 Mikrometer zu durchstechen;
b) jede der Mikroprojektoren (10) umfaßt eine Basis, einen Eindringanteil (15) mit einer Länge L2, eine Spitze (14), eine Kante, eine Fläche und eine Länge L1, das heißt ein Abstand von der Basis bis zur Spitze (14) des Mikroprojektors, wobei die Länge deutlich länger als die vorbestimmte Eindringtiefe ist;
und **dadurch gekennzeichnet, daß**:
c) ein Eindringtiefenbegrenzer (12) an ein oder mehrere der Mikroprojektoren (10) angegliedert ist und an einer vorbestimmten Stelle zwischen der Spitze (15) und der Basis angeordnet ist, wobei der Begrenzer (12) das Durchstechen der Mikroprojektoren (10) auf ungefähr die vorbestimmten Tiefe einschränkt.

**2.** Vorrichtung nach Anspruch 1, wobei der Begrenzer (12) eine Haut-Anstoßfläche umfaßt.

**3.** Vorrichtung nach Anspruch 2, wobei die Haut-Anstoßfläche des Begrenzers (12) zum Zeitpunkt, wenn die Vorrichtung die Haut durchsticht, annähernd parallel zur Hautoberfläche ist.

**4.** Vorrichtung nach Anspruch 2, wobei der Abstand zwischen der Spitze (14) des Mikroprojektors (10) und der Haut-Anstoßfläche des Begrenzers (12) im Wesentlichen der vorbestimmten Tiefe gleicht.

**5.** Vorrichtung nach Anspruch 1, wobei der Begrenzer (12) eine integrale Struktur von einem oder mehrerer der Mikroprojektoren (10) ist.

6. Vorrichtung nach Anspruch 5, wobei der Begrenzer (12) eine integrale Struktur von jedem Mikroprojektor (10) ist.

7. Vorrichtung nach Anspruch 6, wobei jeder Mikroprojektor (10) eine Mehrzahl an Begrenzern (12) hat.

8. Vorrichtung nach Anspruch 5, wobei der Begrenzer (12) eine Haut-Anstoßfläche ist.

9. Vorrichtung nach Anspruch 8, wobei mindestens 10% der Mikroprojektoren (10) mindestens einen Begrenzer (12) haben.

10. Vorrichtung nach Anspruch 1, wobei der Begrenzer (12) eine vom Eindringanteil (15) von einem oder mehreren der Mikroprojektoren (10) ausgezogene Schulter umfaßt.

11. Vorrichtung nach Anspruch 8, wobei annähernd alle Mikroprojektoren (10) mindestens einen Begrenzer (12) haben.

12. Vorrichtung nach Anspruch 1, wobei die Vorrichtung ein mittelenthaltendes oder mittelempfangenes Reservoir umfaßt.

13. Vorrichtung nach Anspruch 12, wobei das Reservoir in einer mittelübertragenden Beziehung mit den Öffnungen (18) in dem Blatt (16) steht.

14. Vorrichtung nach Anspruch 1, wobei das erste Element ein Blatt (16) mit einer Vielzahl an Öffnungen (18) darin einschließt und die Mikroprojektoren (10) aus der körperseitigen Oberfläche des Blatts (16) ausgezogen sind.

15. Vorrichtung nach Anspruch 14, wobei der Begrenzer ein zweites Element (108) mit einer Vielzahl an Stoppvorsprüngen (112) umfaßt, wobei der Begrenzer an die hautabgewandte Seite des Blatts (116) angrenzend angeordnet ist und die Vorsprünge (112) passend beabstandet sind, um sich in die Öffnungen (118) des Blatts (116) zu erstrecken, wobei sich die Stopvorsprünge (112) auf einem vorbestimmten Abstand durch die Öffnungen (118) des Blatts (116) erstrecken.

16. Vorrichtung nach Anspruch 15, wobei die Mikroprojektoren (110) an den Öffnungen (118) angrenzend angeordnet sind, durch welche sich die Stopvorsprünge (112) erstrecken.

17. Vorrichtung nach Anspruch 15, wobei die Länge des Mikroprojektors (110) abzüglich des vorbestimmten Abstandes der Vorsprungsausdehnung (112) annähernd der vorbestimmten Eindringtiefe gleicht.

18. Vorrichtung nach Anspruch 15, wobei das Element (108) mit einer Vielzahl an Stopvorsprüngen (112) aus einem von der aus Plastik, Elastomer und Gummi bestehenden Gruppe ausgewähltem Material besteht.

19. Vorrichtung nach Anspruch 1, wobei das Verhältnis von L2 zu L1 geringer als ungefähr 0,5 ist.

20. Vorrichtung nach Anspruch 14, wobei die Dicke des Eindringanteils (15) von einem oder mehrerer Mikroprojektoren (10) geringer als die Dicke des Blattes (16) ist und der Begrenzer (12) eine oder mehrere an einen oder an mehrere Mikroprojektoren (10) angegliederte hautanstoßende Oberflächen umfaßt.

21. Vorrichtung nach Anspruch 20, wobei die Breite des Blattes (16) abzüglich der Breite des Eindringanteils (15) ungefähr dasselbe ist wie die Breite des Begrenzers (12).


**Revendications**

1. Dispositif pour former plusieurs microcoupures dans la peau d'un animal, les microcoupures ayant une profondeur de pénétration prédéterminée, ledit dispositif comprenant :

   a) un premier élément ayant plusieurs microprojections (10) pour le perçage de la peau s'étendant de celui-ci, comprenant une feuille (16) ayant une surface proximale de peau et une surface distale de peau, la feuille (16) ayant une multiplicité d'ouvertures (18) dans celle-ci, et les microprojections (10) s'étendent d'une surface proximale de corps de la feuille (16), les microprojections (10) étant aptes à percer la peau à une profondeur de pénétration prédéterminée inférieure à environ 500 microns ;

b) chacune des microprojections (10) comprenant une base, une portion de pénétration (15) d'une longueur L2, une pointe (14), un bord, une face et une longueur L1 qui est une distance de la base à la pointe (14) de la microprojection, ladite longueur étant sensiblement plus longue que ladite profondeur prédéterminée ;
et **caractérisé en ce que** :
c) un limiteur de profondeur de perçage (12) est associé à une ou plusieurs des microprojections (10) et est positionné à un emplacement prédéterminé entre la pointe (15) et la base, où le limiteur (12) limite le perçage des microprojections (10) à environ la profondeur prédéterminée.

2. Dispositif selon la revendication 1, où le limiteur (12) comprend une surface butant contre la peau.

3. Dispositif selon la revendication 2, où la surface butant contre la peau du limiteur (12) est approximativement parallèle à la surface de la peau au moment où le dispositif pénètre dans la peau.

4. Dispositif selon la revendication 2, où la distance entre une pointe (14) de la microprojection (10) et la surface butant contre la peau du limiteur (12) est sensiblement égale à la profondeur prédéterminée.

5. Dispositif selon la revendication 1, où le limiteur (12) est une structure intégrale d'une ou de plusieurs microprojections précitées (10).

6. Dispositif selon la revendication 5, où le limiteur (12) est une structure intégrale de chaque microprojection (10).

7. Dispositif selon la revendication 6, où chaque microprojection (10) présente plusieurs limiteurs (12).

8. Dispositif selon la revendication 5, où le limiteur (12) est une surface butant contre la peau.

9. Dispositif selon la revendication 8, où au moins 10% des microprojections (10) possèdent au moins un limiteur (12).

10. Dispositif selon la revendication 1, où le limiteur (12) comprend un épaulement s'étendant depuis ladite portion de pénétration (15) d'une ou de plusieurs desdites microprojections (10).

11. Dispositif selon la revendication 8, où sensiblement toutes les microprojections (10) possèdent au moins un limiteur (12).

12. Dispositif selon la revendication 1, où le dispositif comprend un réservoir contenant un agent ou recevant un agent.

13. Dispositif selon la revendication 12, où le réservoir est en relation de transmission d'agent avec les ouvertures (18) dans la feuille (16).

14. Dispositif selon la revendication 1, où le premier élément comprend une feuille (16) ayant une multiplicité d'ouvertures (18) dans celle-ci, et les microprojections (10) s'étendent d'une surface proximale de corps de la feuille (16).

15. Dispositif selon la revendication 14, où le limiteur comprend un deuxième élément (108) ayant une multiplicité de saillies d'arrêt (112), le limiteur étant positionné d'une manière adjacente au côté distal de la peau de la feuille (116), les saillies (112) étant espacées d'une manière appropriée pour s'étendre dans les ouvertures (118) de la feuille (116), les saillies d'arrêt (112) s'étendant sur une distance prédéterminée à travers les ouvertures (118) dans la feuille (116).

16. Dispositif selon la revendication 15, où les microprojections (110) sont positionnées d'une manière adjacente aux ouvertures (118) à travers lesquelles s'étendent les saillies d'arrêt (112).

17. Dispositif selon la revendication 15, où la longueur de la microprojection (110) moins la distance prédéterminée de l'extension de la saillie (112) est sensiblement égale à la profondeur de pénétration prédéterminée.

18. Dispositif selon la revendication 15, où l'élément (108) ayant une multiplicité de saillies d'arrêt (112) est constituée d'un matériau sélectionné dans le groupe constitué de plastique, élastomère et caoutchouc.

19. Dispositif selon la revendication 1, où le rapport de L2 à L1 est inférieur à environ 0,5.

**20.** Dispositif selon la revendication 14, où l'épaisseur de ladite portion de pénétration (15) d'une ou de plusieurs microprojections (10) est inférieure à l'épaisseur de la feuille (16), et ledit limiteur (12) comprend une ou plusieurs surfaces butant contre la peau associées à une ou plusieurs microprojections (10).

**21.** Dispositif selon la revendication 20, où la largeur de la feuille (16) moins la largeur de la portion de pénétration (15) est environ la même que la largeur du limiteur (12).

**FIG. 1**
**(Prior Art)**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5810857 A
- US 6197039 B
- US 6091975 A, Cormier
- US 6219574 B
- US 6230051 B, Godshall
- US 5879326 A
- WO 0141863 A, Trautman
- US 5820562 A
- WO 0230309 A1, Cormier
- WO 0230301 A1, Cormier